# EUROPEAN PATENT APPLICATION

(11) **EP 4 379 039 A1**
(43) Date of publication of application: **05.06.2024**
(21) Application number: 22849244.3
(22) Date of filing: 12.07.2022
(51) Int. Cl.: C12M 1/34, G06T 7/00, G06T 7/571, G01N 33/483, G01N 21/17

(54) **IMAGE PROCESSING METHOD, COMPUTER PROGRAM, AND RECORDING MEDIUM**

(30) Priority: 28.07.2021 JP 2021123128
(71) Applicant: SCREEN Holdings Co., Ltd., Kyoto-shi, Kyoto 602-8585 (JP)
(72) Inventor: TOKISUE, Shogo, Kyoto-shi, Kyoto 602-8585 (JP); OKAMOTO, Satoshi, Kyoto-shi, Kyoto 602-8585 (JP); OGI, Hiroshi, Kyoto-shi, Kyoto 602-8585 (JP); FURUTA, Tomoyasu, Kyoto-shi, Kyoto 602-8585 (JP)
(74) Representative: Goddar, Heinz J.
(86) International application number: PCT/JP2022/027419
(87) International publication number: WO 2023/008180

(57) **Abstract**

An image processing method according to the invention includes obtaining a plurality of original images at mutually different depths of focus captured by imaging the specimen, generating a composite image including images of the specimen included in each of the plurality of original images in one image plane, and inputting the composite image to a classification model constructed in advance and obtaining an output of the classification model. The classification model is constructed by performing machine learning in advance using teacher images including a plurality of images of same cell or cell mass and having mutually different depths of focus in one image plane. It is possible to obtain useful information on the specimen automatically from a plurality of images obtained by imaging the specimen including a cell at mutually different depths of focus.

## Description

### [Technical Field]

This invention relates to an image processing method for analyzing an image obtained by imaging a specimen including a cell.

### [Background Art]

In the technical fields of pathological medicine and cell culture, it is necessary in some cases to judge the quality, a developmental status and the like of three-dimensionally cultured cells, tissue sections and the like. For such a purpose, so-called multi-focus images captured by an optical microscope at different depths of focus with respect to a specimen in multiple stages may be, for example, used. This is because, since cells have optical transparency, clear images of the specimen at various depths can be obtained by imaging at different depths of focus.

In each image of this case, a clear image is obtained for a structure near a focus position in the specimen. On the other hand, an image of a structure at a position distant from the focus position is unclear and blurry. Thus, to grasp the entire specimen having a three-dimensional structure, it is necessary to observe a multitude of images and make a comprehensive judgment.

An image processing technique for reducing a burden of such an observation operation has also been proposed. For example, in a technique described in PTL 1, whether an image of a tissue to be observed is normal or abnormal is determined by inputting the image to a classification model. More specifically, some images are selected from a cross-sectional image group (called a "3D volume" in PTL 1) obtained for normal tissues in advance. Then, morphological characteristics of the tissues are obtained in the selected images and a classification model is constructed by machine learning. The classification model constructed in this way has a function of determining whether an object to be observed is normal or abnormal based on how many characteristics of the normal tissues the object has.

### [Citation List]

### [Patent Literature]

[PTL 1] JP 2020-032190A

### [Summary of Invention]

### [Technical Problem]

The above conventional technique is based on clear differences of morphological characteristics between two attributes such as normal tissues and abnormal tissues. Thus, this technique is valid only for classification between two attributes. Further, since the classification depends only on information obtained from case examples biased to specific attributes, versatility is not necessarily high. Further, there is also a problem that only some of those pieces of data are effectively utilized even if many case examples are collected.

From these, it is required to establish a technique capable of automatically obtaining information useful for analysis and diagnosis such as the number, quality, types and the like of cells from multi-focus images obtained by imaging a specimen having a three-dimensional structure. Particularly, it is convenient to automatically obtain such information that cannot be obtained unless the multi-focus images are comprehensively evaluated.

### [Solution to Problem]

This invention was developed in view of the above problem and aims to provide a technique capable of automatically obtaining information on a specimen from a plurality of images obtained by imaging a specimen including cells at mutually different depths of focus.

To achieve the above aim, one aspect of this invention is directed to an image processing method for analyzing a specimen including a cell and the image processing method includes obtaining a plurality of original images at mutually different depths of focus captured by imaging the specimen, generating a composite image including images of the specimen included in each of the plurality of original images in one image plane, and inputting the composite image to a classification model constructed in advance and obtaining an output of the classification model. Here, the classification model is constructed by performing machine learning in advance using teacher images including a plurality of images of same cell or cell mass at mutually different depths of focus in one image plane.

In the invention thus configured, a predetermined output is obtained by giving an input image to the classification model constructed by machine learning in advance. Here, a relationship of the input image and the output obtained in response to the input image, i.e. which output is derived from the classification model having the input image given thereto, can be specified by how teaching data corresponding to the teacher image is given in the stage of machine learning. In this invention, which output is obtained from the classification model does not matter. However, the input image to be input to the classification model and the teacher image applied to machine learning to construct the classification model are as follows.

First, an image to be input to the classification model is a composite image generated to include images of a specimen respectively included in a plurality of original images (i.e. multi-focus images) obtained by imaging the specimen including a cell at mutually different depths of focus in one image plane. A plurality of the images obtained by imaging one specimen at various depths of focus are included in this composite image. Each of those images clearly represents the structure of the specimen near the depth of focus. Therefore, the entire composite image has information on the detailed structure of the specimen at various depths.

A teacher image is also an image including a plurality of images of the same cell or cell mass and having mutually different depths of focus in one image plane. Therefore, one entire teacher image includes images obtained when the cell or cell mass is imaged at various depths of focus and has information on the detailed structures of the cell or cell mass at various depths.

The classification model constructed by machine learning performed using such teacher images returns an output derived from the information on the detailed structures at various depths of focus, which information is possessed by each of the images of the specimen included in the composite image. This means nothing more or less than the realization of the aforementioned function of "comprehensively evaluating the multi-focus images and automatically obtaining information". That is, according to the invention, useful information on the specimen can be automatically obtained from a plurality of images obtained by imaging the specimen at mutually different depths of focus.

Further, another aspect of this invention is directed to a computer program for causing a computer to perform each step of the image processing method described above. Further, still another aspect of this invention is directed to a computer-readable recording medium non-transitorily recording the computer program. In the invention thus configured, an existing computer device can be, for example, used as an execution subject of the invention.

### [Advantageous Effects of Invention]

As described above, according to the invention, a composite image obtained by arranging images of a specimen captured at mutually different depths of focus in the same plane is used as an input image, and the input image is input to a classification model constructed in advance using similar images as teacher images. Thus, it is possible to obtain a result output comprehensively evaluating the structure of the specimen having a three-dimensional structure.

The above and further objects and novel features of the invention will more fully appear from the following detailed description when the same is read in connection with the accompanying drawing. It is to be expressly understood, however, that the drawing is for purpose of illustration only and is not intended as a definition of the limits of the invention.

### [Brief Description of Drawings]

[FIG. 1] FIG. 1 is a diagram showing a schematic configuration of an imaging apparatus capable of performing an image processing method according to the invention.
[FIG. 2A] FIG. 2A is a diagram schematically showing the structure of an embryo serving as a biological specimen in this embodiment.
[FIG. 2B] FIG. 2B is a diagram schematically showing the structure of an embryo serving as a biological specimen in this embodiment.
[FIG. 3] FIG. 3 is a flow chart summarizing the cell counting process of this embodiment.
[FIG. 4] FIG. 4 is a diagram schematically showing an example of a tiled image.
[FIG. 5A] FIG. 5A is a drawing showing a method for constructing a classification model.
[FIG. 5B] FIG. 5B is a drawing showing a method for constructing a classification model.
[FIG. 6] FIG. 6 is a chart illustrating relationships of teacher images and teaching inputs.
[FIG. 7] FIG. 7 is a diagram schematically showing a process of generating a tiled image from an image stack.
[FIG. 8] FIG. 8 is a chart showing examples of the rule for selecting the original images from the image stack.
[FIG. 9] FIG. 9 is a flow chart showing a modification of the cell counting process.

### [Description of Embodiments]

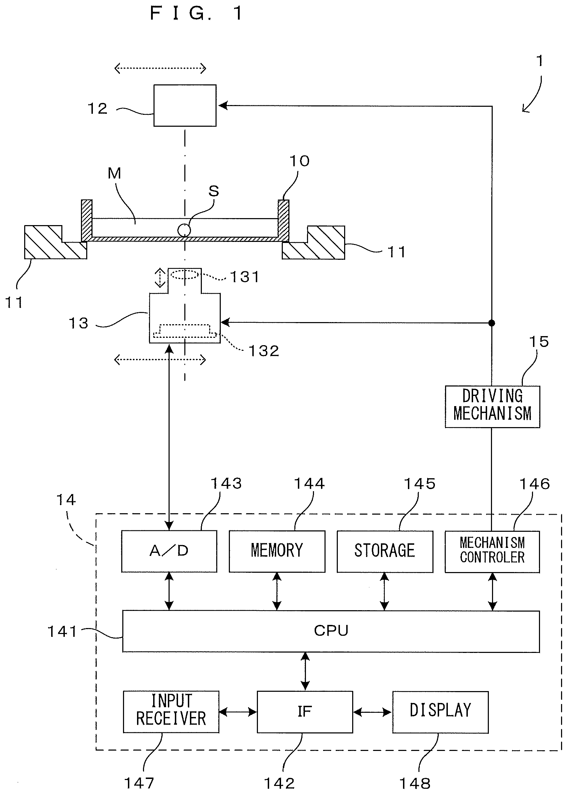

FIG. 1 is a diagram showing a schematic configuration of an imaging apparatus capable of performing an image processing method according to the invention. This imaging apparatus 1 is an apparatus for imaging a biological specimen S such as cells C carried in a specimen container (generally called a dish) 10 having a flat shape and an open upper surface. A predetermined amount of a liquid serving as a culture medium M is injected into the specimen container 10. The cells or the like cultured under predetermined culture conditions in this liquid serve as an imaging object of this imaging apparatus 1. The culture medium may be added with an appropriate reagent or may be gelled after being injected in a liquid state into the specimen container 10.

Note that a case where a biological specimen is used as the imaging object is illustrated here. However, the imaging object of the invention is not limited to this. For example, a tissue section, a pathological specimen or the like carried in an appropriate carrier may be the imaging object. Further, the specimen container may be a well plate provided with a plurality of recesses referred as "well" and a biological specimen carried in each well may be the imaging object.

The imaging apparatus 1 includes a holder 11 which holds the specimen container 10, an illuminator 12 arranged above the holder 11, an imager 13 arranged below the holder 11 and a controller 14 which includes a CPU 141 controlling the operation of these components. The holder 11 holds the specimen container 10 in a substantially horizontal posture by being held in contact with a peripheral edge part of the lower surface of the specimen container 10.

The illuminator 12 emits an illumination light toward the specimen container 10 held by the holder 11. For example, a white LED (light emitting diode) may be used as a light source of the illumination light. A combination of the light source and an appropriate illumination optical system are used as the illuminator 12. The imaging object in the specimen container 10 is illuminated by the illuminator 12 from above.

The imager 13 is provided below the specimen container 10 held by the holder 11. In the imager 13, an imaging optical system is arranged at a position right below the specimen container 10 and an optical axis of the imaging optical system extends in a vertical direction. FIG. 1 shows a side view and an up and down direction of the figure indicates a vertical direction.

By the imager 13, a bright-field imaging of the imaging object in the specimen container 10 is performed. Specifically, as follows. Light emitted from the illuminator 12 and incident on the surface of the liquid from above the specimen container 10 illuminates the imaging object. Light transmitted downward from the bottom surface of the specimen container 10 is incident to a light receiving surface of an imaging element 132 via the imaging optical system of the imager 13 including an objective lens 131. An image (bright-field image) of the imaging object formed on the light receiving surface of the imaging element 132 by the imaging optical system is imaged by the imaging element 132. The imaging element 132 is an area image sensor having a two-dimensional light receiving surface and, for example, a CCD sensor or a CMOS sensor can be used as the imaging element 132.

The imager 13 is capable of moving in the horizontal direction and the vertical direction by a mechanism controller 146 provided in the controller 14. Specifically, the mechanism controller 146 moves the imager 13 in the horizontal direction by operating a driving mechanism 15 based on a control command from the CPU 141. By doing so, the imager 13 moves relative to the specimen container 10 in the horizontal direction. Further, focusing is performed by moving the objective lens 131 in the vertical direction.

As indicated by arrows with dotted lines shown in FIG.1, the driving mechanism 15 moves the illuminator 12 integrally with the imager 13 when the imager 13 is moved in the horizontal direction. Specifically, the illuminator 12 is arranged such that a center of emitted light substantially coincides with the optical axis of the imager 13. When the imager 13 moves in the horizontal direction, the illuminator 12 also moves in conjunction with the imager 13. By doing so, whenever the imager 13 moves relative to the specimen container 10, the center of light emitted from the illuminator 12 always positions on the optical axis of the imager 13. Consequently, the illuminating condition becomes constant regardless of which specimen container 10 is to be imaged, wherefore imaging conditions can be maintained to be satisfactory.

The image signal output from the imaging element 132 of the imager 13 is send to the controller 14. That is, the image signal is input to an AD converter (A/D) 143 provided in the controller 14 and converted into digital image data. The CPU 141 performs appropriate image processings based on the received image data.

The controller 14 further includes a memory 144 for temporarily storing image data and calculation result and a storage 145 for storing programs to be executed by the CPU 141 and data generated by the CPU 141. The memory 144 can be accessed from the CPU 144 at high speed, but has less storage capacity than the storage 145. The storage 145 includes a hard disk drive (HDD), for example, which has more storage capacity and less access speed than the memory 144. These can be used properly according to the purpose. The CPU 141 performs variable calculation processings described later by executing a control program stored in the storage 145.

Besides, the controller 14 is provided with an interface (IF) 142. The interface 142 has a function of receiving an operation input from a user and presenting information such as processing results to the user. The controller 14 also has a function of performing data exchange with an external apparatus connected via a communication line. To realize the user interface function, an input receiver 147 for receiving an operation input from the user and a display 148 for displaying the messages to the user, a processing result or the like are connected to the interface 142.

In this example, imaging is performed by causing illumination light to be incident from above the biological specimen S in the specimen container 10 and receiving the light transmitted downward. For example, bright field imaging using an inverted optical microscope is, in principle, similar to this. However, conversely to the above, imaging may be performed by causing illumination light to be incident from below the specimen and receiving the light transmitted upward. For example, bright field imaging using an erect optical microscope falls under this.

One embodiment of an image processing method according to the invention executable using the imaging apparatus 1 configured as described above is described below. As described later, the imaging apparatus 1 has a function of imaging the biological specimen S while changing a focus position in multiple stages in an optical axis direction of the objective lens 131, i.e. the vertical direction (z direction). In this way, an image set composed of a plurality of images obtained by imaging the biological specimen S at various different depths of focus, i.e. a so-called multi-focus image stack, is generated. In the following description, when the "focus position" is merely referred to, it means the focus position in a depth direction, i.e. the z direction, unless otherwise specified. Further, the focus position in the depth direction may be merely referred to as a "focus depth".

The imaging apparatus 1 further has a function of deriving qualitative or quantitative information on the type, state and the like of the biological specimen S having a three-dimensional structure by performing a later-described image processing using the multi-focus image stack. This image processing includes class classification using a classification model constructed in advance by machine learning. Various proposals have been thus far made for a specific method for analyzing a biological specimen using a machine-learned classification model and which attribute of a biological specimen is set as a class classification object. Those can be appropriately selected and applied also in this imaging apparatus 1.

Here, a cell counting process is described in which a fertilized egg (hereinafter, merely referred to as an "embryo") in an initial stage of development is imaged as the specimen S and the number of cells constituting this embryo is counted from the captured images. An embryo evaluation operation by a user (specifically, an expert such as a doctor or embryologist) can be effectively supported based on data obtained by this. For example, in the culture of an embryo for the purpose of a fertility treatment, a cell counting method of this embodiment can be applied for the purpose of obtaining knowledge for judging whether or not the culture is satisfactorily in progress. Note that an object to be processed is not limited to such an embryo, and this process is applicable to various cell masses in which a plurality of cells are closely in contact.

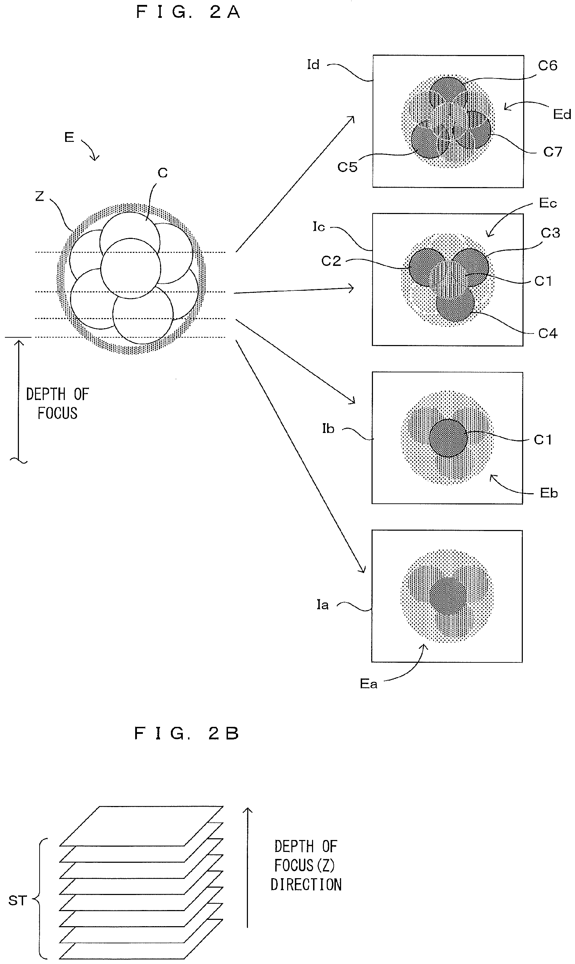

FIGS. 2A and 2B are diagrams schematically showing the structure of an embryo serving as a biological specimen in this embodiment. As already known, if an egg is fertilized, cleavage starts and a blastocyst is formed after a state called a morula. The cell counting method of this embodiment is suitable for counting the number of cells constituting an embryo, for example, until a morula stage immediately after fertilization.

FIG. 2A schematically shows the structure of the embryo in an initial stage (e.g. from a 4-cell stage to the morula stage). The embryo E has a substantially spherical outer shape. The surface of the embryo E is covered with a layer Z of a jelly glycoprotein called a zona pellucida. A plurality of cells C produced by the cell division of the fertilized egg are included inside. In a state where the culture is satisfactorily in progress, the inside of the zona pellucida Z is taken up by relatively large cells C as shown in a left figure of FIG. 2A. As the cleavage proceeds, the number of the cells C increases.

If such an embryo E is imaged in a bright field while a depth of focus indicated by a dotted line in FIG. 2A is variously changed, a plurality of images schematically shown in right figures of FIG. 2A are obtained. In an image Ia having a smallest depth of focus when viewed from the imager 13 arranged below the specimen container 10, i.e. having a focus position positioned on a lowermost side, none of the cells C constituting the embryo E is focused and the image Ia is an unclear image.

In an image Ib in a state where the focus position is moved upward and, for example, the lowest cell C1 is focused, this cell C1 is clear. In an image Ic obtained by moving the focus position further upward, the cell C1 no longer in focus becomes unclear and the cells C2 to C4 at positions close to the focus position appear as clearer images. If the cell C1 is nearly transparent, other cells located to the back of the cell C1 appear in the image through the cell C1. In an image Id obtained by moving the focus position further upward, the cells C1 to C4 no longer in focus become unclear and images of the upper cells C5 to C7 at positions closer to the focus position appear through the cells C1 to C4.

An image of the embryo E included in the image Ia is denoted by Ea, an image of the embryo E included in the image Ib is denoted by Eb, an image of the embryo E included in the image Ic is denoted by Ec and an image of the embryo E included in the image Id is denoted by Ed below.

In this way, the same specimen is imaged in the same field of view a plurality of times while the depth of focus is changed and set in multiple stages. By doing so, as shown in FIG. 2B, a set of a plurality of original images having the same field of view and mutually different depths of focus can be obtained. This image set is called an image stack (more particularly, a multi-focus image stack). In each image of the image stack ST, images of the cells at positions close to the focus position at the time of imaging, out of the cells constituting the cell mass (in this example, the embryo E), are clear images. On the other hand, the images of the cells distant from the focus position are unclear. Further, the images of the cells to the back of the cells in front of the focus position (closer to the imager 13) appear through those front cells. In the cell counting process of this embodiment, the number of the cells constituting the embryo is counted, utilizing such characteristics of the image stack ST.

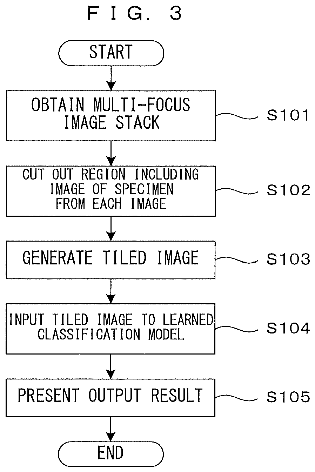

FIG. 3 is a flow chart summarizing the cell counting process of this embodiment. This process and each process described below are realized by the CPU 141 reading out and implementing a control program stored in advance in the storage 145 and causing each component of the apparatus to perform a predetermined operation. Note that these various processes can be realized also by a computer device having a general hardware configuration except the process of imaging the biological specimen S. Thus, this embodiment may be realized by a combination of an imaging device having an imaging function and a computer device having an image processing function. Further, if an already captured image stack is present, an image processing using that image stack can be realized also by the computer device.

First, bright field imaging is performed at mutually different depths of focus for the biological specimen S (here, the embryo E) as an object to be processed a plurality of times. In this way, a plurality of images are obtained and a multi-focus image stack ST is generated (Step S101). Images may be newly obtained by the imager 13 performing imaging or image data obtained in advance by imaging and stored in an external storage or the like may be obtained from outside through an appropriate memory medium or an electric telecommunication line. Out of the plurality of obtained images, at least some images are used as "original images" in the following image processing.

As understood from FIG. 2A, in each individual image in the image stack ST, a clear image is obtained for the cell present near the focus position and includes detailed information on the shape and structure of this cell. However, an image is unclear for the cell at a position distant from the focus position and it is difficult to obtain detailed information.

Accordingly, for the purpose of counting the number of the cells constituting the embryo E, it is necessary to specify and count the individual cells not from a single image but comprehensively from a plurality of images having different depths of focus. However, a classification model for returning one of a plurality of class classifications for one input image cannot necessarily be said to be suitable for such a purpose.

Accordingly, in this embodiment, partial regions including the image of the embryo E are cut out from the plurality of images constituting the image stack ST (Step S102). A composite image is generated by arranging those partial regions in one image plane (Step S 103). This composite image is used as an input image for the classification model. A processing for generating one image by combining a plurality of images or partial regions cut out from the plurality of images is called "tiling". A composite image generated in this way is referred to as a "tiled image" below.

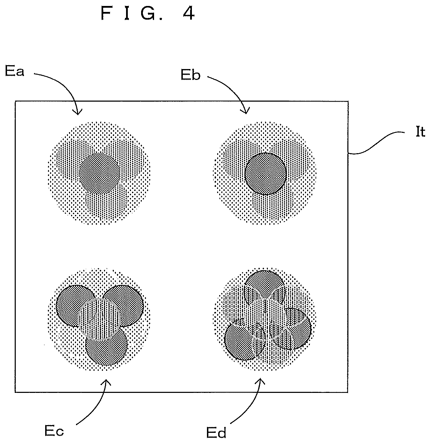

FIG. 4 is a diagram schematically showing an example of a tiled image. In this tiled image It, out of the plurality of images constituting the image stack ST, the image Ea of the embryo E cut out from the image Ia, the image Eb of the embryo E cut out from the image Ib, the image Ec of the embryo E cut out from the image Ic and the image Ed of the embryo E cut out from the image Id are arranged in a single image plane. In such a tiled image It, the images of the embryo E captured at various depths of focus are included in one image. Therefore, this image includes information representing the structure of the embryo E at various depths.

As just described, a tiled image including a plurality of images having mutually different depths of focus and, therefore, including information on various depths is input as an input image to the already learned classification model (Step S104). Although specifically described later, the classification model is constructed in advance by supervised learning using "images each including a plurality of images having mutually different depths of focus" similarly to the input image as teacher images. The classification model analyzes information included in the input image and outputs a corresponding classification class based on a learning result.

An output result is presented in an appropriate mode to the user (Step S105). For example, by displaying an output of the classification model or a determination result based on this output on the display unit 148, a processing result can be presented to the user. In this example, the number of the cells constituting the embryo E serving as the biological specimen S is output as a result. In this way, the number of the cells constituting the embryo E is counted from the image stack ST obtained by the multi-focus imaging of the embryo E.

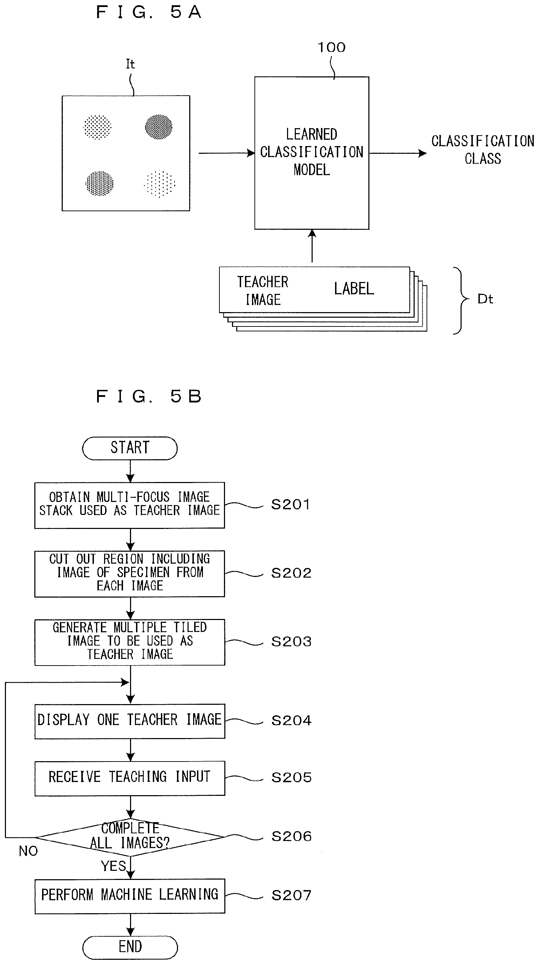

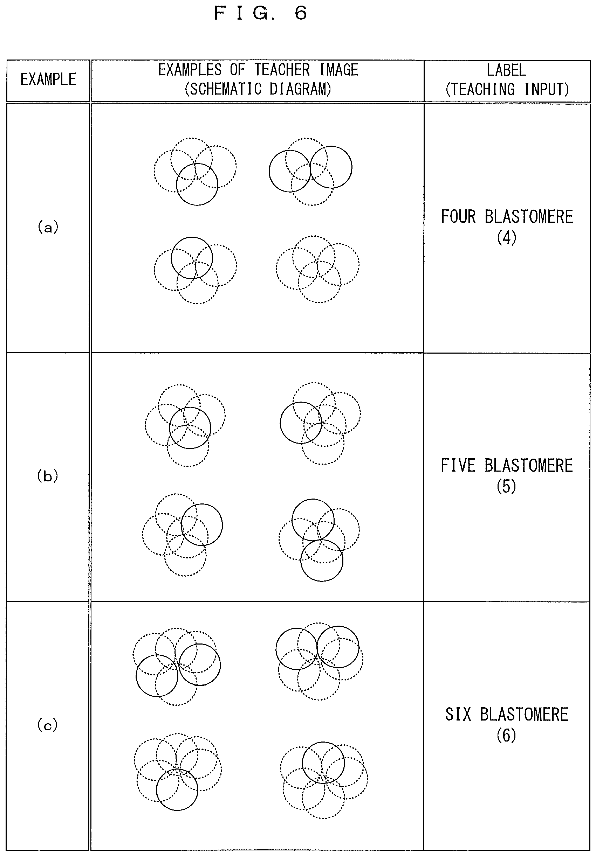

FIGS. 5A and 5B are drawings showing a method for constructing a classification model. More specifically, FIG. 5A is a diagram schematically showing the classification model. Further, FIG. 5B is a flow chart showing a process for allowing the classification model to be learned. Further, FIG. 6 is a chart illustrating relationships of teacher images and teaching inputs.

As shown in FIG. 5A, a classification model 100 has a function of receiving an input of the tiled image It generated from the image stack ST obtained by imaging the embryo E to be analyzed and outputting the number of the cells constituting this embryo E. To enable this, a multitude of pieces of teacher data Dt associating the teacher image and a label as the teaching input representing a classification class to be given to the teacher image are collected and machine learning using those is performed.

Specifically, as shown in FIG. 5B, a multi-focus image stack including a plurality of original images used as raw materials of a teacher image is first obtained (Step S201). For example, such an image stack can be obtained by imaging a biological specimen of the same type as the biological specimen S (embryo E in this example) to be analyzed while changing a depth of focus in multiple stages. All or some of the images included in the image stack become "original images" used in the following processing.

Then, a region including the image of the biological specimen is cut out from each original image (Step S202), and one teacher image is generated by tiling for synthesizing those regions. A plurality of teacher images are generated (Step S203). As a method for generating a plurality of teacher images, for example, a plurality of biological specimens can be prepared and Steps S201 to S203 can be performed for each of those biological specimens. Further, a plurality of image sets each obtained by selecting several images from a multitude of images obtained by imaging one biological specimen may be prepared and a teacher image may be generated from each of the image sets.

Out of the teacher images generated in this way, one teacher image is displayed on the display unit 148 (Step S204). Then, a teaching input from the user on a label representing a classification class to be given to the displayed teacher image is received (Step S205). In this example, the number of the cells constituting the embryo E can be set as the label.

In a schematic diagram of the teacher image shown in FIG. 6, it is assumed that a solid-line circle represents the cell imaged in a focused state and a dotted-line circle represents the cell imaged in an unfocused state, i.e. in a blurry state. Among the plurality of images of the embryo included in the teacher image, the cell(s) in the focused state are different from each other due to differences in depth of focus. The user observes the plurality of images included in the displayed teacher image, reads the number of the cells constituting this embryo and inputs that result as a teaching.

In a case example shown in (a) of FIG. 6, it is difficult to read the number of the cells only from the individual images included in the teacher image, but four blastomeres composed of four cells can be judged if the plurality of images are comprehensively looked. The user inputs a label "4" as a teaching for this teacher image. Similarly, a label "5" is taught for a case example (b) of five blastomeres, and a label "6" is taught in a case example (c) of six blastomeres. It is desirable to collect as many case examples as possible, including case examples having as many cells as these and having different numbers of the cells.

Steps S204 to S205 are repeatedly performed until the teaching input is completed for all the teacher images (Step S206). In this way, the labels are given to all the teacher images. Then, machine learning is performed based on these teacher images (Step S207). In this manner, the classification model 100 is constructed.

Various classification algorithms trained by supervised learning can be applied as a classification algorithm for realizing the classification model 100. From the perspective of widely utilizing various pieces of information obtained from images, a deep learning algorithm requiring no artificial designation of characteristics to be extracted is suitable. For example, a convolutional neural network can be applied.

The classification model 100 constructed in this way acquired a function of analyzing the images of the embryo E captured at various depths of focus and included in the input image and outputting the number of the cells constituting this embryo E. Further, by changing the label to be given to each teacher image at the time of machine learning, another function can be realized. For example, by giving labels on various attributes such as a growth stage of an embryo, the types and shapes of cells constituting a biological specimen and the quality of a cultured state and learning, those attributes can be automatically determined from the images.

Next, a more specific method for generating a tiled image from a multi-focus image stack is described. A way of thinking described below is similarly applicable to both a case where a tiled image serving as a teacher image is generated and a case where a tiled image serving as an input image to a classification model is generated.

First, as to the image stack ST, to obtain information from each of the plurality of cells included in the biological specimen S, all the cells are desirably shown as clear images at least in one image. Accordingly, a feed pitch of the focus position at the time of imaging is desirably set to be nearly equal to or smaller than the size (specifically, a length in the z direction) of each cell constituting the biological specimen. For example, if the size of the cells is about 10 µm, the focus pitch can be set to be nearly equal to this or several µm slightly smaller than this. Note that if a depth of field of the imaging optical system is relatively larger than the size of the cells, the feed pitch of the focus position may be slightly larger.

Then, the image stack ST generated to entirely cover the biological specimen in the depth direction possibly includes a multitude of (e.g. several tens of) images. It is not realistic to tile all the images of the biological specimen cut out from those into one composite image. This is because image data of the composite image becomes very large and the subsequent image processing becomes complicated. Further, there may be no large difference in obtained information among images having near depths of focus.

From this, for the image stack ST including a multitude of images, it is realistic to select an appropriate number of the images from these images, cut out the images of the biological specimen included in those images and synthesize the cut-out images as a tiled image. Further, by doing so, a plurality of tiled images can be generated from one image stack ST by making a combination of the images to be selected different. This helps to enrich case examples serving as teachers in generating teacher images. Further, in generating an input image to the classification model, the input image can be used to evaluate the certainty of an output result of the classification model as described later.

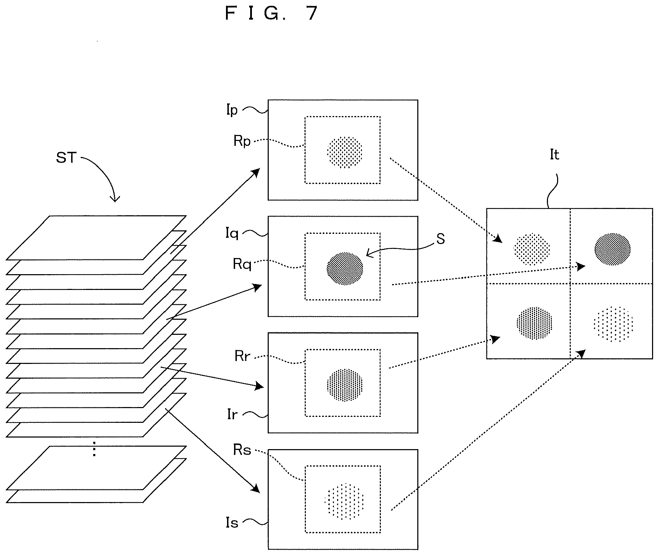

FIG. 7 is a diagram schematically showing a process of generating a tiled image from an image stack. Several original images Ip, Iq, Ir and Is are selected in an appropriate selection rule from the image stack ST including the multitude of original images obtained by the multi-focus imaging of the biological specimen S. The original images selected in this way include images of the biological specimen S having different focused states, therefore, having mutually different clarities. A region Rp, Rq, Rr, Rs including the image of the biological specimen S is cut out from each image Ip, Iq, Ir, Is. The tiled image It is generated by combining those regions in the same image plane.

The tiled image It includes a plurality of the images of the biological specimen S cut out from the respective original images, and those images have different focused states. Therefore, the tiled image It includes information representing the structure of the biological specimen S at various depths.

The tiled image It may be synthesized, directly using the selected images Ip, Iq, Ir and Is as the original images. However, by cutting out and using partial regions of the respective images, it is possible to remove in advance such image objects that might become obstructive factors in the later image processing such as a wall surface of the culture container, air bubbles included in the culture liquid and image noise. Further, this also contributes to a reduction in image data size.

The image selection rule is arbitrary. For example, the images can be extracted at certain intervals in the order of the focus position, but the intervals may not necessarily be equal intervals. Particularly, in generating the tiled image serving as a teacher image, the user may check each image and artificially extract the preferable images as typical examples. For example, it is possible to exclude images including no clear image of the cell and images with much noise. Note that it does not mean that these images are not suitable as teacher images, and these images can be, for example, used as typical examples corresponding to such irregular situations. In short, in selecting the images from the image stack ST, various rules may be applied according to a purpose.

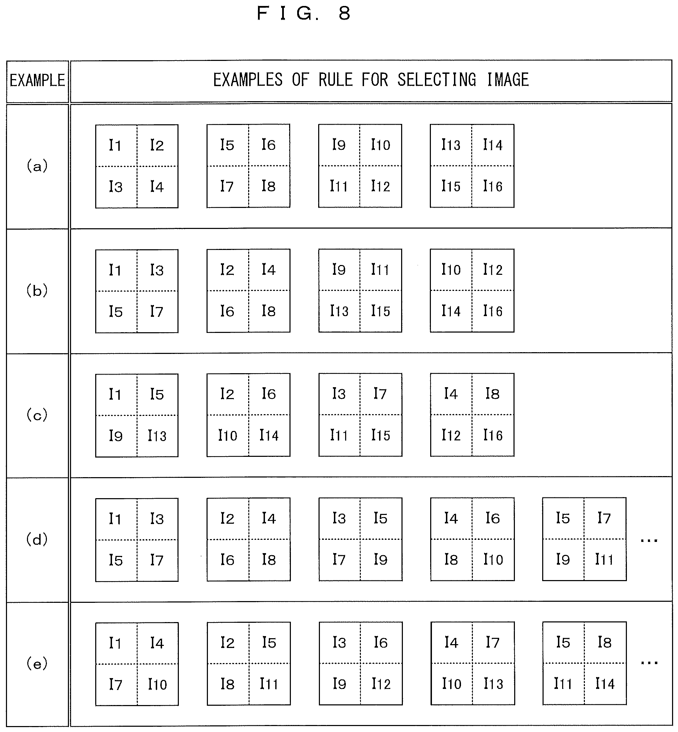

FIG. 8 is a chart showing examples of the rule for selecting the original images from the image stack. Here, it is assumed that the image stack ST includes sixteen images and those images can be distinguished from each other by giving reference signs I₁, I₂, I₃, ..., I₁₆ along the order of the focus position. Further, one tiled image It is assumed to include the images of the biological specimen S cut out from four original images. However, these numerical values are merely examples for description and there is no limitation to this.

A case example (a) is an example of a simplest selection rule, four images are selected each time from the images I₁ to I₁₆ in the order of the focus position and set as original images, and a total of four tiled images are generated. In this case, there is a large deviation in information in the depth direction reflected on each tiled image. That is, a range in the depth direction reflecting the information of the biological specimen is largely different between the tiled image composed of the original images I₁ to I₄ and the tiled image composed of the original images I₁₁ to I₁₆. Thus, mutual correlation may not be high.

For the purpose of comprehensively evaluating the entire biological specimen, it is desirable to reflect information in as wide a depth range as possible in one tiled image. From this perspective, every other original image is selected in the case example (b) and every four original images is selected in the case example (c) from the image stack ST. By doing so, information in a wider depth range can be included in each tiled image.

On the other hand, the case example (d) is similar to the case example (b) in that one tiled image is generated by selecting four original images I₁, I₃, I₅ and I₇ every other one with one image, e.g. the image I₁ as a starting point. However, unlike the case example (b) in which one original image is reflected only in one tiled image, the images I₁, I₂, I₃, ... serving as starting points are successively shifted by one in the case example (d), whereby one original image is included in a plurality of tiled images. A combination of the original images is different in each tiled image. Another case example (e) is different from the case example (d) in that every three images are selected, but the same way of thinking is applied.

As just described, the following effect is obtained by including one original image in a plurality of tiled images while changing a combination of the original images. First, in generating a teacher image, the accuracy of machine learning can be improved by diversifying and enriching case examples serving as teachers. Further, in generating an input image to a classification model, the reliability of an output result can be improved. The reason for that is as follows.

In the case of selecting several images as original images from an image stack and generating a tiled image, information included in the tiled image is not all information possessed by the image stack, but is biased to information possessed by the selected images. Thus, depending on a combination of the original images included in an input image, a classification result by a classification model is possibly different. This causes a reduction in the reliability of the result.

If a plurality of input images having different combinations of the original images are input to the classification model, each output value is thought to be different as a matter of course. However, since the images are originally obtained from the same specimen, the output values do not randomly vary and a certain tendency corresponding to characteristics of this specimen appears in appearance frequencies of various output values. Therefore, such a tendency can be grasped by testing out a variety of combinations for the original images included in the input image.

Specifically, the output value can be said to have a higher probability of being more adapted to the characteristics of the specimen, i.e. being a correct answer, with an increase in the appearance frequency. For example, if ten input images are input to the classification model and seven output values, out of those, match each other, the reliability of that output value can be said to be sufficiently high. On the other hand, two or more output values are obtained with nearly the same frequency, those results can be said to be both low in reliability.

If an output value having a probability of being a correct answer is set as a final output as just described, the reliability of the output result can be improved as compared to the case where a single input image is used. Further, by statistically processing a plurality of output values obtained for a plurality of input images, an index value quantitatively representing the "certainty" of a final output can be derived. Based on this way of thinking, the fundamental cell counting process shown in FIG. 3 can be modified as follows.

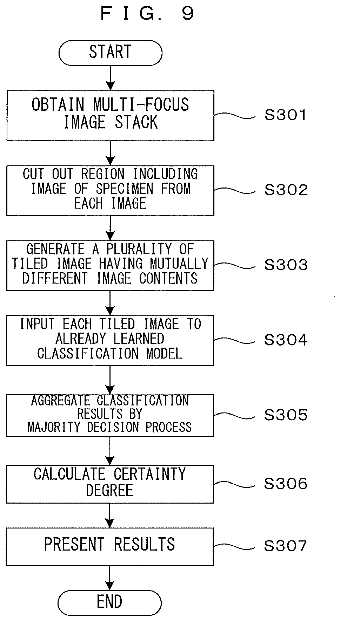

FIG. 9 is a flow chart showing a modification of the cell counting process. This process is the same as Steps S101 to S102 in the process of FIG. 3 in that a multi-focus image stack of the biological specimen S (specifically, the embryo E) to be analyzed is obtained (Step S301) and a region including an image of the biological specimen S is cut out from each image (Step S302). On the other hand, in subsequent Step S303, a plurality of tiled images having mutually different image contents are generated by changing and synthesizing combinations of those regions.

Each of these tiled images is individually input to the classification model 100 (Step S304). In this way, the classification model 100 returns a plurality of outputs, i.e. classification results, corresponding to the respective input images. By statistically processing those, those can be finally aggregated into one classification result.

For example, out of a plurality of classification results corresponding to the plurality of input images, the classification result having a highest appearance frequency can be selected by a majority decision process and set as a final classification result (Step S305). In addition, a value quantitatively indicating the certainty of the final result (here, referred to as a "certainty degree") can be calculated (Step S306).

For example, out of the plurality of output values, a ratio of the output values matching the final result can be set as the certainty degree. For example, if it is assumed that there were seven results of "four blastomeres", two results of "five blastomeres" and one result of "six blastomeres" for ten input images in the classification model for outputting the number of blastomeres of the embryo E, the final result is "four blastomeres" by the majority decision process and the certainty degree thereof is 70 %.

Note that the "certainty degree" represents the certainty of the final result and the certainty degree is defined only for the final result here. As another way of thinking, it is also possible to define a certainty degree for each output value. If this is applied to the above example, a certainty degree for the result of "five blastomeres" is 20 % and a certainty degree for the result of "six blastomeres" is 10 %. According to this way of thinking, a process of setting the output value having a highest certainty degree, out of the respective output values, as the final result is also possible.

Further, it is also possible to obtain the final result and the certainty degree using a softmax function, which is an activation function introduced in the neural network algorithm. Note that since a concept of the softmax function is widely known, description is omitted here.

For example, it is assumed that values of the softmax function with respect to the respective classification classes (four blastomeres to six blastomeres) for three tiled images were:
Image 1 (four blastomeres: 96.9 %, five blastomeres: 2.0 %, six blastomeres: 0.1 %),
Image 2 (four blastomeres: 85.9 %, five blastomeres: 11.0 %, six blastomeres: 1.3 %), and
Image 3 (four blastomeres: 40.3 %, five blastomeres: 51.0 %, six blastomeres: 3.1 %).
Note that since the other classification classes (e.g. seven blastomeres or more, less than four blastomeres) are excluded here, a total value of the softmax function may be less than 100 %.

The classification model outputs the value having a maximum value of the softmax function as the classification result. Therefore, "four blastomeres" is returned as an output for the images 1 and 2 and "five blastomeres" is returned as an output for the image 3. Therefore, the final result by the majority decision process is "four blastomeres" and the certainty degree thereof is 66.7 %.

On the other hand, in the case of obtaining the certainty degree from the softmax function, the following way of thinking may be adopted. Firstly, there is a method for averaging values of the softmax function given to the same classification class as the final result using all the input images as a population. In the above example, since the values of the softmax function given to the classification class of "four blastomeres", which is the final result, are respectively 96.9 %, 85.9 % and 40.3 % in the images 1 to 3, an average value (74.4 %) of these can be set as the certainty degree.

Secondly, there is a method for averaging values of the softmax function given to the same classification class as the final result using only the input images, for which the classification class matching the final result was output, as a population. In the above example, the classification class of "four blastomeres", which is the final result, was output for the images 1 and 2, and an average value (91.4 %) of the softmax function given to these images can be set as the certainty degree.

Further, it is also possible to determine the final result of the classification class based on the softmax function. For example, it is possible to adopt a method for calculating an average value or total value of the softmax function of each classification class for a plurality of input images and setting the classification class having a maximum calculated value as the final result. In the above example, since the average value of the softmax function given to the classification class of "four blastomeres" for the images 1 to 3 is significantly larger than those of the other classification classes, "four blastomeres" can be set as the final result. Further, the average value at this time can be set as the certainty degree of the final result.

As just described, several modes are thought for the definition of the certainty degree. In any mode, it can be said that the larger the certainty degree, the higher the reliability of the final result. That is, the certainty degree mentioned here is a relative value defined among the respective classification classes and the absolute numerical value has no certain quantitative significance. In extreme cases, even if the certainty degree is, for example, 100 %, the correctness of the final result at that time is not perfectly guaranteed. The definition can be appropriately selected depending on how the value of the certainty degree is utilized.

The final result (the number of the blastomeres in this example) of the classification class obtained in the above way and the certainty degree of that result are presented to the user, for example, by being displayed on the display unit 148 (Step S307). By presenting the final classification class and, in addition, the certainty degree thereof, the user can judge how reliable the obtained result is. For example, if the value of the certainty degree is high, it can be judged that the imaging of the biological specimen and classification based on the images obtained thereby were satisfactorily performed. On the other hand, if the value of the certainty degree is low, the user can recognize a possibility of a certain problem in the process thus far such as improper condition setting at the time of imaging.

In such a case, the user can confirm several images included in the image stack and take a necessary measure. For example, measures such as the redoing of a part of the process with the conditions readjusted or the discard of the entire process result are possible.

Further, a utilization method for statistically processing certainty degrees obtained by the cell counting process for each of a plurality of biological specimens and making use of the processing result in the evaluation of this cell counting process itself is also possible. For example, the cell counting process having obtained a certainty degree of a certain value or higher can be judged as a "success", the cell counting process having obtained a certainty degree less than that can be judged as a "failure", and only the result of the succeeded cell counting process can be treated as a valid one.

On the other hand, if a frequency of being judged as a "failure" is high, the conditions of the process such as the setting of the feed pitch of the depth of focus at the time of imaging, the rule in generating the tiled images from the image stack and the like can be revised, whereby a failure frequency can be reduced.

As described above, in this embodiment, a composite image including images of a specimen S captured at various depths of focus in the same image plane is generated from an image stack obtained by the multi-focus imaging of the specimen S including cells to be analyzed such as an embryo E. This composite image is an input image for the classification model 100. The classification model 100 is constructed in advance by performing machine learning using teacher images each including images at various depths of focus similarly to the input image and classification classes corresponding to these teacher images as teacher data.

Accordingly, the classification model 100 has a function of analyzing the information of the specimen at various depths included in the input image and returning the classification class corresponding to the input image as an output. In this embodiment, the embryo E in an initial stage of development is used as the specimen, and the number of the cells constituting the embryo E is used as the classification class. Therefore, the classification model 100 has a function of outputting the number of the cells constituting the embryo E from the multi-focus image stack of the embryo E.

The teacher image and the input image are tiled images including the images of the cells at various depths, and each image exhaustively includes information on the detailed structures of the cells at various depths. Therefore, according to this embodiment, a function of comprehensively evaluating a plurality of images captured at different depths of focus and determining one classification class can be realized for a specimen having a three-dimensional structure. By setting the classification class corresponding to a purpose, various attributes of the specimen can be automatically determined.

Note that the invention is not limited to the embodiment described above and various changes other than the aforementioned ones can be made without departing from the gist of the invention. For example, the imaging apparatus 1 of the above embodiment has itself the imaging function of generating the multi-focus image stack and the image processing function of analyzing the images and performing the cell counting process. However, the cell counting method according to the invention can also be performed by a computer device having no imaging function itself and having obtained an image stack generated by imaging in another apparatus having an imaging function. To enable this, the invention may be carried out as a software (computer program) for causing the computer device to perform each processing step of the above process.

Such a computer program can be distributed, for example, in the form of downloading via an electric telecommunication line such as the Internet. Further, it is also possible to distribute a computer-readable recording medium recording this computer program. Further, by causing, for example, an existing microscope imaging apparatus to read this computer program via an interface, the invention can be carried out by this apparatus.

Further, in the above embodiment, the construction of the machine learning model based on the collected teacher data and the cell counting process utilizing the constructed machine learning model are performed by the same apparatus. However, these may be performed by different apparatuses. For example, the machine learning model may be constructed using a computer device having a high computing power and a classification model as a result may be implemented into an imaging apparatus. By doing so, even the imaging apparatus having a limited computing power can perform the imaging and the cell counting process.

Further, not only the machine learning model constructed by the computer having a high computing power is directly implemented into the imaging apparatus, but also a method called "distillation", that is, a method for inputting the aforementioned input image (tiled image) to the machine learning model constructed in the computer having a high computing power and separately performing machine learning in the imaging apparatus using a combination of an obtained inference result and the input image corresponding to the inference result as learning data may be performed. In this case, the inference result obtained from the machine learning model constructed in the computer having a high computing power corresponds to the aforementioned teaching input.

Further, in the above embodiment, the embryo (fertilized egg) in the initial stage of development is used as the specimen serving as an object to be analyzed of the invention, and it is aimed to automatically count the number of the cells. However, a specimen as an object of the invention and attributes of the specimen sought to be obtained from images of the specimen are not limited to these. For example, even if an embryo is used as a specimen to be analyzed as in the above case, the invention can be applied for the purpose of evaluating the presence or absence of fragments, the amount of the fragments and the like other than the number of the cells. Such an application is possible by changing labels to be given at the time of performing machine learning.

Further, in the evaluation of the embryo, a development stage of the embryo, i.e. in which stage, out of a series of development processes, the embryo is, is determined. It is also possible to apply the invention for the purpose of such a determination. Such a function can be realized by collecting multi-focus image stacks of the embryo in various stages such as an initial embryo, an initial blastocyst stage and an expanded blastocyst stage and performing machine learning by giving labels representing development stages to teacher images generated from those image stacks.

Besides, a processing method similar to the above embodiment can be applied also, for example, for the purpose of judging whether or not the growth of spheroids or organoids artificially produced by cell culturing is good and evaluating medicinal effects of drugs and chemical substances for the spheroids or organoids. As just described, this embodiment is suitable for the purpose of obtaining various pieces of qualitative and quantitative information first obtained by comprehensively evaluating a specimen having a three-dimension structure.

As the specific embodiment has been illustrated and described above, machine learning may be performed based on a plurality of teacher images and one type of classification class taught for each teacher image in the image processing method according to the invention. As a result of learning, the classification model may have a function of outputting the classification class corresponding to inputted composite image. According to such a configuration, a single classification class is obtained as an output for a plurality of images of a specimen obtained at various imaging depths. Therefore, a result comprehensively reflecting the three-dimensional structure of the specimen can be obtained.

Further, for example, one composite image may be generated based on some original images selected from the plurality of original images, a plurality of composite images may be generated by changing a combination of the original images, and one of a plurality of outputs obtained by inputting each of the plurality of composite images to the classification model may be selected as a final output.

In the original images obtained by discretely changing the depth of focus, all information in a depth direction originally possessed by the specimen cannot be reflected. Due to such missing information, an output result of the classification model possibly varies depending on an input image generation method. By synthesizing outputs when a plurality of composite images having different combinations of the original images are used as input images and determining a final output, the bias of the result due to differences of the input image generation method can be reduced.

For example, out of the plurality of outputs when a plurality of composite images are used as input images, the output having a highest appearance frequency can be set as a final output. A variation of outputs is thought not to be random, but to strongly reflect specific attributes originally possessed by the specimen. Therefore, the selection of the output having a highest appearance frequency, out of the plurality of outputs, is a very realistic method.

In these cases, a step of calculating an index value on the certainty of the final output based on the plurality of outputs may be further provided. By calculating such an index value, it is possible to obtain various pieces of incidental information such as the reliability of the obtained result and whether or not the specimen and the processing method are proper.

Here, a classification model obtained, for example, by the deep learning algorithm can be utilized. In the deep learning algorithm, it is not necessary to artificially set characteristics to be extracted from images in advance. If a multitude of case examples suitable as teachers can be collected, an accurate classification model can be constructed. This property is very useful in analyzing specimens having large individual differences and diversified such as cells and tissues including those cells.

Further, the teacher images desirably include images of same type of cell as the cell included in the specimen. As described above, since cells and tissues including the cells have a large diversity, it is effective to perform machine learning using images including the images of the same type of cell as the cell included in the specimen as teacher images to accurately analyze the specimen composed of specific cell.

Further, bright field images of the specimen captured using an optical microscope can be, for example, used as the original images in the invention. In this case, since the cells are nearly transparent to visible light, which is illumination light, the illumination light reaches deep into the inside of the specimen. Information of various depths can be obtained by performing imaging by variously changing a focus position in the depth direction. From this, bright field microscope images are particularly suitable for the purpose of comprehensively analyzing a specimen having a three-dimensional structure.

Further, in the invention, the specimen is, for example, an embryo and the classification model can output the number of cells included in the embryo. For example, if the classification model is constructed by performing machine learning based on a plurality of teacher images generated based on images of embryos having mutually different numbers of blastomeres and classification classes represented by taught values as the numbers of blastomeres corresponding to the respective teacher images, the number of the cells constituting the embryo can be automatically counted.

Although the invention has been described with reference to specific embodiments, this description is not meant to be construed in a limiting sense. Various modifications of the disclosed embodiment, as well as other embodiments of the present invention, will become apparent to persons skilled in the art upon reference to the description of the invention. It is therefore contemplated that the appended claims will cover any such modifications or embodiments as fall within the true scope of the invention.

### [Industrial Applicability]

This invention is suitable for the purpose of analyzing a specimen including a cell by an image processing and particularly suitable for an application required to comprehensively evaluate the structure of a specimen having a three-dimensional structure. For example, in the field of assisted reproductive medicine, an operation of evaluating a state of a cultured embryo (fertilized egg) can be effectively supported.

### [Reference Signs List]

- 1: imaging apparatus
- 10: specimen container
- 13: imager
- 14: controller
- C: cell
- E: embryo (specimen)
- Ia-Id, Ip-Is: original image
- It: tiled image
- S: specimen
- ST: image stack

## Claims

1. An image processing method for analyzing a specimen including a cell, the image processing method comprising:
obtaining a plurality of original images at mutually different depths of focus captured by imaging the specimen;
generating a composite image including images of the specimen included in each of the plurality of original images in one image plane; and
inputting the composite image to a classification model constructed in advance and obtaining an output of the classification model, wherein
the classification model is constructed by performing machine learning in advance using teacher images including a plurality of images of same cell or cell mass at mutually different depths of focus in one image plane.

2. The image processing method according to claim 1, wherein:
the machine learning is performed based on a plurality of the teacher images and one type of classification class taught for each teacher image; and
the classification model outputs the classification class corresponding to inputted composite image.

3. The image processing method according to claim 1 or 2, wherein:
the composite image is generated based on the original images selected from the plurality of the original images and a plurality of the composite images are generated by changing a combination of the original images; and
one of a plurality of outputs obtained by inputting each of the plurality of composite images to the classification model is selected as a final output.

4. The image processing method according to claim 3, wherein out of the plurality of outputs, one output having a highest appearance frequency is the final output.

5. The image processing method according to claim 3 or 4, further comprising calculating an index value on a certainty of the final output based on the plurality of outputs.

6. The image processing method according to any one of claims 1 through 5, wherein the classification model is constructed by a deep learning algorithm.

7. The image processing method according to any one of claims 1 through 6, wherein the teacher images include images of same type of cell as the cell included in the specimen.

8. The image processing method according to any one of claims 1 through 7, wherein the original images are bright field images of the specimen captured using an optical microscope.

9. The image processing method according to any one of claims 1 through 8, wherein the specimen is an embryo and the classification model outputs number of cells included in the embryo.

10. The image processing method according to claim 9, wherein the machine learning is performed based on: a plurality of the teacher images generated based on images of embryos having mutually different numbers of blastomeres; and classification classes represented by taught values as the numbers of blastomeres corresponding to the respective teacher images.

11. A computer program, configured to cause a computer device to perform each processing of the image processing method according to any one of claims 1 through 6.

12. A computer-readable recording medium, storing non-transitorily the computer program according to claim 11.
